# EUROPEAN PATENT APPLICATION

(11) **EP 2 161 042 A1**
(43) Date of publication of application: **10.03.2010**
(21) Application number: 09175610.6
(22) Date of filing: 27.01.2006
(51) Int. Cl.: A61L 27/18, A61L 27/50, A61F 2/12

(54) **A Prosthesis and Method of Manufacturing a Prosthesis**

(30) Priority: 29.01.2005 ZA 200408765; 29.01.2005 ZA 200410188; 20.04.2005 ZA 200503184; 27.05.2005 ZA 200504333; 22.08.2005 ZA 200506704; 14.09.2005 ZA 200507390; 21.10.2005 ZA 200508552; 17.11.2005 ZA 200509314
(62) Divisional of application: 06710272.3
(71) Applicant: Smart Implant PLC, 3 Fleet Street London EC4Y 1DP (GB)
(72) Inventor: Hamilton, Jonathan, 2040, Honeydew (ZA)
(74) Representative: Giles, Ashley Simon

(57) **Abstract**

A prosthesis is disclosed which includes a filler material comprising a biocompatible granular substance. The prosthesis includes in a particular embodiment a human mammary prosthesis. The granular substance comprises a mixture of synthetic biocompatible cells and a lubricant. Also disclosed is an osmotic barrier which reduces the flow of lubricant from the prosthesis and the flow of body fluids into the prosthesis.

## Description

### FIELD OF THE INVENTION

This invention relates to a prosthesis and a method of manufacturing a prosthesis in particular but not limited to a mammary prosthesis.

### BACKGROUND TO THE INVENTION

Mammary prostheses are used to enhance the appearance of human breasts, normally female breasts. The prostheses are used to add volume to a breast which may be required after removal of a tumour, or to enhance the aesthetic appeal of a breast.

A mammary prosthesis typically comprises a resilient plastics material bladder which is filled with a liquid. The bladder is normally manufactured from a silicone rubber filled with a gel, typically a silicone gel.

A problem with silicone gel is rupturing of the bladder which releases the gel in the patient's body. If a bladder ruptures *in vitro* the released gel cannot be recovered, or at least not all of it.

A serious complication of silicone gel filled bladder prostheses is capsular contraction, which is a condition in which tissue surrounding the prosthesis after it has been implanted into a patient hardens. This hardening of the tissue takes place in response to silicone gel which routinely leaks through the bladder. The leaking is an accepted side effect of these prostheses and capsulation is, in part, relied upon to contain silicone which leaks from the bladder.

One likely driving force for the bleed of the filler material, and specifically silicone gel, is Brownian motion, which is a result of thermal molecular motion of particles in a liquid environment. According to this phenomenon, there is random movement of the silicone gel particles inside the shell of the prosthesis. Since the atomic structure of solid silicone, from which the shell of the prosthesis is manufactured, is larger than the size of silicone gel molecules, some of the gel molecules will pass through the shell because of the Brownian motion. In essence, the shell is like a mesh through which the gel particles can be squeezed.

Another phenomenon which also likely acts as a driving force is osmotic pressure. According to Merriam-Webster's Collegiate Dictionary, osmosis is the "movement of a solvent through a semi-permeable membrane (as of a living cell) into a solution of higher solute concentration that tends to equalize the concentrations of solute on the two sides of the membrane." In the case of a silicone gel filled mammary prosthesis, there is a higher concentration of gel inside the shell of the prosthesis than outside the shell. Body fluid, of which there is plenty in supply, will tend to move into the prosthesis because of osmotic pressure in an attempt to equalize the concentrations of silicone gel on opposite sides of the shell. This increases the fluid pressure inside the prosthesis which contributes as a driving force to push fluids out of the prosthesis, including silicone gel. At the same time the silicone gel, which is at a higher concentration inside the shell than in the human body and is at least to some degree a solute, experiences osmotic pressure to move from a higher concentration to a lower concentration through the shell, i.e. from the prosthesis into the body.

There have been attempts to limit the spread of the silicone by formulating it as a 'sticky' gel. The intention with this is to keep the silicone gel together and to ease surgical removal. However, this does not solve the problem since the sticky gel adheres to anything it touches including the patient's organs and the surgeon's gloves. Removing the sticky gel is not an easy matter and 100% removal of all leaked gel is generally not possible.

In some countries, the use of bladders filled with silicone gel has been banned due to the health risks associated with it. To overcome this problem prostheses have been developed which comprise a bladder filled with liquid other than silicone gel, for example saline solution. This solves the problem of the leaking which causes capsular contraction. If such a prosthesis ruptures the patient doesn't need surgery to remove the liquid, since saline is harmless to the human body.

A problem associated with saline filled prostheses is that these prostheses are generally inserted empty into a patient and filled *in vitro* by means of a filler tube and non return valve. These systems are often problematic and leakage of saline through the filler tube arrangement often occurs.

Another problem with saline filled prostheses is that the viscosity of saline is different from silicone gel, which causes these prostheses to have an unnatural feel once implanted.

Another problem with conventional prostheses is that details of implanted prostheses are not readily ascertainable from outside the body. In some instances it is necessary to determine details, such as size and type of implant or the date on which the prostheses was implanted on short notice and preferably without surgical procedure. Such instances may include a medical emergency such as may arise following an accident. It may also occur during routine procedures.

It often happens that people are unable to convey details of a prosthesis to medical personnel which may leave the medical personnel with no option but to determine details of the implanted prosthesis by means of expensive scanning equipment, for example MRI scanning, or surgery. Neither of these is desirable, the first due to the cost involved and second due to the invasive and drastic nature and the cost thereof.

A specific problem exists with female patients whom had received breast augmentation surgery and experience complications. In many instances, these patients are not able to recall the make of prosthesis they have received and in even more cases, not the size of prosthesis received. If an existing prosthesis needs to be replaced the surgeon needs to have all possible sizes available during surgery to fit the correct size prosthesis.

A further aspect of breast prostheses that is problematic relates to the manufacturing of the prostheses. In most cases a bladder is formed which include an opening at the operatively posterior side of the bladder. This opening is needed in the forming of the bladder to enable removal from the mould on which the bladder is formed. The opening is sealed by means of a disc which is placed inside the opening and adhered to the bladder by means of pressure. A problem with this type of seal is that it is not a seal that is formed by means of a bond between the two surfaces forming part of the seal, but rather an adhesion type seal of the disc against the bladder which, after the bladder has been filled, relies on pressure from the gel inside the bladder to maintain the seal. It is possible to disengage such a seal by exerting on the disc from the outside.

The above mentioned problems have been exemplified by way of a mammary prosthesis, but similar problems exist with other types of prostheses. Examples of these include prostheses which are used to enhance the appearance of buttocks, cheeks, and biceps. The concerns about the safety of silicone gel filled prostheses are equally applicable to these procedures, as are problems which exist with saline filled prostheses.

### OBJECT OF THE INVENTION

It is an object of the invention to provide a prosthesis which at least partly overcomes the abovementioned problem.

### SUMMARY OF THE INVENTION

In accordance with this invention there is provided a human prosthesis comprising a resilient shell at least partly filled with a biocompatible granular substance, and preferably for the prosthesis to comprise a human mammary prosthesis.

There is further provided for the granular substance to comprise a plurality of biocompatible synthetic cells, preferably cells manufactured from a resilient material, and preferably for the resilient material to comprise a medical grade silicone.

There is further provided for a plurality of micro glass balloons to be dispersed in each cell, preferably between 2% and 10%, further preferably between 4% and 8%, still further preferably between 5% and 7%, and more preferably about 6% micro glass balloons by volume.

There is further provided for the micro-balloons to comprise commercially available micro-balloons with a diameter of about 1 micron each.

There is also provided for each cell to have a lenticular shape, alternatively an ovoid or a bean shape and for the shell to be filled, at least partly, with cells having a range of sizes.

According to a preferred embodiment of the invention each cell having a lenticular shape has a circular diameter of about 6mm and a height of about 2mm.

According to a further feature of the invention there is provided for each cell to comprise a hollow capsule encapsulating a fluid, preferably a biocompatible fluid, further preferably oil, still further preferably a vegetable oil, and yet further preferably soy bean oil; alternatively a biocompatible gas, further preferably air, still further preferably sterilized air.

There is still further provided for each hollow cell to have a wall thickness of about 0.1 mm.

A further feature of the invention provides for each cell to include at least one tracer adapted to be detectable by means of external diagnostic equipment.

There is further provided for the cells to be located within a lubricant, preferably a biocompatible lubricant.

According to a still further feature there is provided for the shell of the prosthesis to include a filling aperture, for the aperture to comprise a recessed portion in the shell, preferably a circular recess, and for a complimentary circular seal to be securable in the recess to seal the shell.

There is still further provided for the circular seal to be securable to the shell by means of glue, preferably silicone glue.

There is still further provided for the recess to include a right circular cylindrical outer wall and for the seal to include a complimentary right circular cylindrical circumference.

There is still further provided for the seal to comprise a membrane and a plug.

There is further provided for the membrane to be securable to the operative base of the recess and for the plug to be securable onto the membrane in the recess.

There is still further provided for the membrane to have a thickness of about 0.1 mm and for the plug to have a thickness of about 0.9mm.

There is also provided for the plug and the membrane to be manufactured from the same material as the shell, preferably a medical grade silicone material.

There is also provided for the shell surrounding the recess to be thickened such that the operatively inner surface of the shell inside of the aperture is substantially step-less.

The invention also provides for the mammary prosthesis to comprise a shell which contains a filler material, for the shell to comprise at least one resilient layer and at least one layer being substantially non-permeable with respect to at least the filler material of the shell; and for the filler material to comprise, preferably a biocompatible granular substance, alternatively a fluid filler material, in particular a silicone gel or a saline based fluid.

There is further provided for the resilient layer to comprise a sealed operatively outer container, for the non-permeable layer to comprise a sealed operatively inner container which contains the filler material and for the inner container to be contained by the outer container, and preferably for the prosthesis to include a lubricant between the inner container and outer container.

There is still further provided for the non-permeable layer to be non-permeable also with respect to body fluids.

There is still further provided for the surface area of the inner container to be greater than the surface area of the outer container, and preferably at least twice as large, alternatively for the surface area of the inner container to be at least as great as the maximum surface area to which the outer container may be stretched elastically.

According to a further feature of the invention there is provided for the non-permeable layer to comprise a composite layer, and for the composite layer to comprise a nylon layer located between a polyester and polyethylene layer.

There is still further provided for the lubricant within which the cells are located to inlcude a hydrophilic polymer, preferably a water soluble polymer or hydrogel, and further preferably polyvinyl pyrrolidone (PVP), and for the non-permeable layer to include at least a polyethylene layer.

There is further provided for the hydrophilic polymer to be mixed with a saline solution, and preferably for the lubricant to comprise about 15% hydrophilic polymer and about 85% saline solution.

There is still further provided for the prosthesis in one embodiment to have a volume between 200 cm³ and 450 cm³ and to include the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 82.352 | 82.352 |
| 225 | 92.646 | 92.646 |
| 250 | 102.94 | 102.94 |
| 275 | 113.234 | 113.234 |
| 300 | 123.528 | 123.528 |
| 325 | 133.822 | 133.822 |
| 350 | 144.116 | 144.116 |
| 375 | 154.41 | 154.41 |
| 400 | 164.704 | 164.704 |
| 425 | 174.998 | 174.998 |
| 450 | 185.292 | 185.292 |

There is also provided for the prosthesis in a further embodiment to have a volume between 200 cm³ and 450 cm³ and to include the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 87.5 | 87.5 |
| 225 | 98.4375 | 98.4375 |
| 250 | 109.375 | 109.375 |
| 275 | 120.3125 | 120.3125 |
| 300 | 131.25 | 131.25 |
| 325 | 142.1875 | 142.1875 |
| 350 | 153.125 | 153.125 |
| 375 | 164.0625 | 164.0625 |
| 400 | 175 | 175 |
| 425 | 185.9375 | 185.9375 |
| 450 | 196.875 | 196.875 |

There is also provided for the prosthesis in a yet further embodiment to have a volume between 200 cm³ and 450 cm³ and to include the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 95 | 95 |
| 225 | 106.875 | 106.875 |
| 250 | 118.75 | 118.75 |
| 275 | 130.625 | 130.625 |
| 300 | 142.5 | 142.5 |
| 325 | 154.375 | 154.375 |
| 350 | 166.25 | 166.25 |
| 375 | 178.125 | 178.125 |
| 400 | 190 | 190 |
| 425 | 201.875 | 201.875 |
| 450 | 213.75 | 213.75 |

According to further feature of the invention there is provided for the non-permeable layer to comprise a composite layer which comprises at least a composite polyethylene and aluminium layer, for the lubricant to include glycerine, and preferably for the lubricant to comprise a combination of water and glycerine.

There is still further provided for the for the aluminium layer to be a vapour deposited layer on the polyethylene layer and for the vapour deposited aluminium layer to have a thickness in the range of about 12 micron to 18 micron.

There is also provided for the non-permeable layer to have a thickness of between about 20 micron and about 70 micron, preferably between about 40 micron and 55 micron, more preferably to have a thickness of about 48 micron.

According to a yet further feature of the invention there is provided for the granular substance which fills the prosthesis to be contained in an inner container, preferably manufactured from a biocompatible resilient mesh.

According to a still further feature of the invention there is provided for the outer container to contain a plurality of inner containers, for each of the plurality of inner containers to contain a plurality of cells, and for each inner container to comprise a non-permeable layer including a nylon layer located between a polyester and polyethylene layer and within which the plurality of cells are located in a hydrophilic polymer lubricant; alternatively for each inner container to comprise a non-permeable layer including at least a composite polyethylene and aluminium layer within the plurality of cells are located in a lubricant which includes glycerine, preferably a lubricant which comprises a combination of water and glycerine.

According to a yet further feature of the invention the shell has a predetermined shaped corresponding to that of a breast.

According to an alterative feature of the invention there is provided for the tracer to comprise a metal particle, preferably a titanium particle, contained in the cell.

There is also provided for the prosthesis to include identification means bearing data relating to at least the size of the prosthesis, preferably the number of cells contained within the prosthesis, and further preferably for the identification means to comprise an identification tab on which the data is printed.

According to a further feature of the invention, there provided for the prosthesis to include a remotely activatable data transmission device configured to transmit a predetermined data carrying signal in response to remote activation thereof.

There is further provided for the transmission device to comprise a data transponder, preferably in the form of a Radio Frequency Identification (RFID) tag, and for the RFID to be remotely activatable by means of an energy field, preferably a magnetic field.

There is still further provided for the prosthesis to comprise a human mammary prosthesis, for the prosthesis to comprise a biocompatible resilient container filled with a biocompatible granular substance and at least one data transmission device, preferably an RFID tag.

There is still further provided for the RFID tag to be incorporated into a cell of the granular substance, alternatively for the RFID tag to be incorporated into the shell, further alternatively to be incorporated into the filler opening seal of the shell.

There is also provided for the prosthesis to be manufactured in a range of sizes and for each size to include a predetermined number of cells.

In accordance with a further feature of the invention there is provide a method of manufacturing cells for use in a human mammary prosthesis as defined above, the method comprising coating two plates, which each has a plurality of cavities therein and in which each cavity corresponds to a semi-portion of a cell, with a liquid resilient material, arranging the two plates to align each individual cavity of one plate with another individual cavity of the other plate, allowing the resilient material to set, removing the plates from each other, and removing cells formed between the formations from the plates.

There is also provided for the method to include placing a tracer in one semi-portion of each cell prior to arranging the plates together.

There is still further provided for the method to include trimming excess resilient material located around the circumference of each cell, and preferably for the excess material to be trimmed by integral trimming formations in at least one of the plates, each trimming formation surrounding a half-portion cavity and being shaped and configured to press against the surface of the opposing plate upon aligning of the plates to trim excess resilient material from the formed cell.

According to a further feature of the invention there is provided a method of filling a shell for a human mammary prosthesis with a filler material, comprising the steps of securing a complimentary shaped membrane to an aperture in the shell, inserting a needle into the shell through the membrane, injecting a predetermined amount of a liquid biocompatible lubricant through the needle into the shell, retracting the needle, and sealing the filler aperture with a complimentary shaped seal.

There is also provided for the method to include the step of filling the shell with a plurality of biocompatible resilient cells, prior to securing the membrane to the filler aperture, and thereafter injecting a predetermined amount of the liquid lubricant through the needle, preferably a glycerine and water mixture.

There is further provided for a method of filling a shell of a human mammary prosthesis which includes the steps of filling an inner container, as defined above, with a predetermined number of biocompatible synthetic cells and a predetermined volume of liquid lubricant, sealing the inner container, inserting the inner container into an outer container, as defined above, securing a complimentary shaped membrane to an aperture in the shell, inserting a needle into the shell through the membrane, injecting a predetermined amount of a liquid biocompatible lubricant through the needle into the shell, retracting the needle, and sealing the filler aperture with a complimentary shaped seal.

There is also provided for the method to include evacuating substantially all air from the shell prior to injecting the lubricant into the shell.

### BRIEF DESCRIPTION OF THE DRAWINGS

Embodiments of the invention are described by way of example only and with reference to the accompanying drawings in which:
- Figure 1: is a sectional view of a first embodiment of a human mammary prosthesis according to the invention;
- Figure 2: is a sectional view of a biocompatible synthetic cell of the prosthesis of Figure 1;
- Figure 3: is a sectional view of the prosthesis of Figure 1 implanted into a female breast;
- Figure 4: is shows a sectional view of a second embodiment of a human mammary prosthesis according to the invention which includes an inner container;
- Figure 5: is a sectional view of a first embodiment of a laminate used for the inner container of the prosthesis of Figure 4;
- Figure 6: is a sectional view of a second embodiment of layers used for the inner and outer containers of a human mammary prosthesis according to the invention;
- Figure 7: is a sectional view of a second embodiment of layers used for the inner and outer containers of a human mammary prosthesis according to the invention;
- Figure 8: is a sectional view of a third embodiment of a human mammary prosthesis according to the invention;
- Figure 9: is a sectional view of a fourth embodiment of a human mammary prosthesis according to the invention;
- Figures 10 to 14: are sectional views showing one embodiment of a method of filling a human mammary prosthesis according to the invention;
- Figure 15: is a sectional view of a mould used to manufacture biocompatible synthetic cell according to the invention; and
- Figure 16: shows detail of a part of the mould of Figure 15.

### DETAILED DESCRIPTION OF THE INVENTION

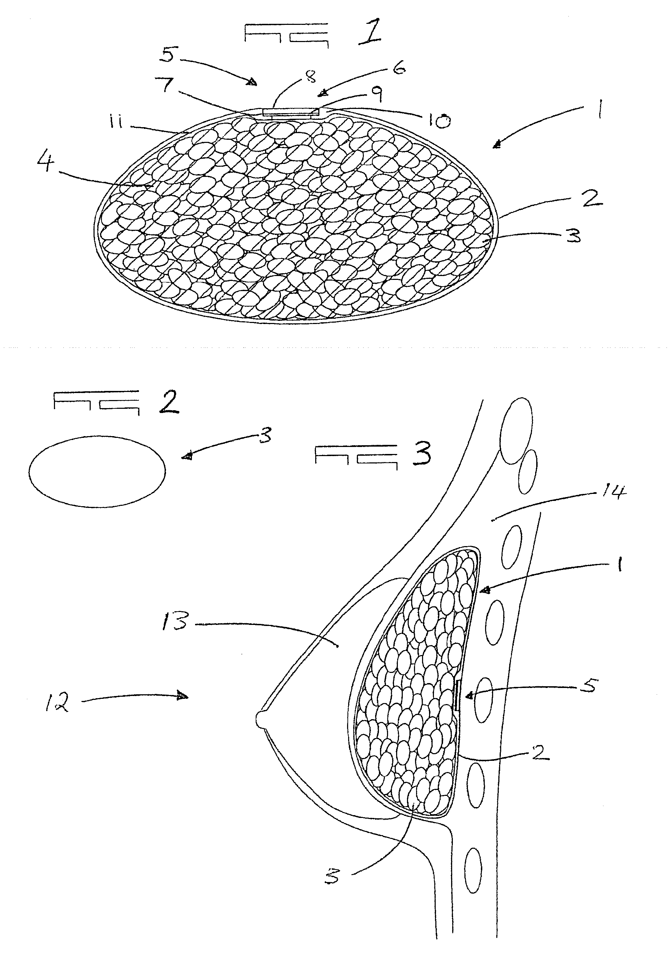

A first embodiment of a human mammary prosthesis (1) according to the invention is shown in the drawings. The prosthesis (1) shown in Figure 1 comprises a resilient shell (2) filled with a granular substance which in this embodiment is a plurality of cells (3). The shell (2) also includes a lubricant (4) amongst the cells (3).

The cells (3) are manufactured from a biocompatible synthetic and resilient material, namely a silicone rubber. As shown in Figure 2 each cell (3) has a lenticular shape having a circular diameter of about 6 mm and a height of about 2 mm.

Each cell (3) includes a plurality of micro glass balloons (not shown) dispersed throughout it. These hollow micro glass balloons are minute in size, each measuring only about one micron in diameter. The micro balloons are mixed into the silicone rubber from which the cells (3) are manufactured and remains dispersed therein. This means the micro glass balloons cannot migrate from the cells (3).

The micro glass balloons are included at about 6% by volume, although it has been established that inclusion of micro balloons in the range of between 2% and 10%, between 4% and 8%, and between 5% and 7% also yields acceptable results.

The micro balloons are included in the cells (3) to render the cells (3) visible during medical examination. The medical grade silicone rubber, from which the cells (3) are manufactured, are normally relatively transparent, not only to the human eye but also to X-rays. The inclusion of the micro glass balloons renders the cells (3) opaque and visible during a breast examination. This is important since an examining physician may want to be certain that he does not confuse the cells (3) of the prosthesis (1) with a lump in the breast. By making the cells (3) visible under an X-ray the physician can clearly identify the cells (3).

The inclusion of the micro glass balloons also serves another purpose. In the event of the prosthesis (1) rupturing and some of the cells (3) escaping from the shell (2), the cells (3) may be located easily by making use of an X-ray or MRI scanner.

The prosthesis (1) includes a filling aperture (5) which is closed with a circular seal (6). The filling aperture (5) comprises a circular recessed portion (7) in the shell (2). The seal (6) is circular and is securable in the recess (7) to seal the shell (2). The seal (6) is secured to the shell (2) by means of a silicone glue.

The seal (6) includes two layers, which includes a membrane (8) and a plug (9). The membrane (8) is first secured the shell (2) at the base of the recess (7), after which the plug (9) is secured onto the membrane (8). The membrane (8) and plug (9) are manufactured from the same medical grade silicone rubber as the shell (2) and the cells (3).

As can be seen in Figure 1 the shell (2) is thickened (10) in the area surrounding the recess (7) such that the operatively inner surface (11) of the shell (2) around the aperture (5) is substantially step-less. This is to keep the inner surface (11) of the shell (2) smooth to ensure unimpeded movement of the cells (3) inside the shell (2).

In use the prosthesis (1) is implanted in the normal manner into a female patient's breast (12), as shown in Figure 3. The prosthesis (1) is surgically implanted using normal surgical techniques. In this embodiment, the prosthesis (1) is implanted underneath the breast tissue (13) in the pectoral muscle (14), but it may also be implanted above the pectoral muscle (,14) as is commonly done in human mammary prosthesis procedures.

The prosthesis (1) is implanted with the filling aperture (5) and seal (6) at the operatively back of the prosthesis (1), to eliminate the chance of this thicker portion of the prosthesis (1) being felt through the breast tissue (13) of the patient.

The prosthesis (1) provides more of a natural feel to the breast (12) than conventional saline or gel filled prostheses do. This is due to the lubricated silicone cells (3) which are free to move around inside the shell (2) and which mimics natural breast tissue.

The prosthesis (1) is manufactured to a specific predetermined size, and includes a known predetermined number of cells (3).

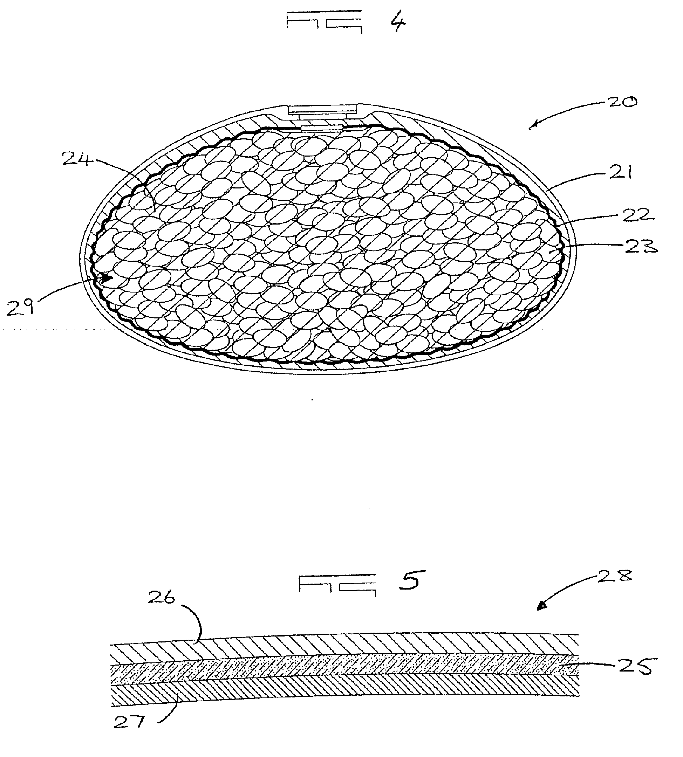

A second embodiment of a human mammary prosthesis (20) according to the invention is shown in Figure 4. This prosthesis (20) includes a resilient shell (21), which forms an outer container, and within which an inner container (22) is located. The inner container (22) contains a plurality of the biocompatible synthetic cells (23), as shown in Figure 2, and a lubricant (24).

The inner container (22) of the prosthesis of Figure 4 comprises a laminate, as shown in Figure 5, of a nylon layer (25) which is sandwiched between polyester layer (26) and a polyethylene layer (27). This composite inner layer (28) which forms the inner container (22) is non-permeable to at least the filler material (29) of the prosthesis (20) and body fluids of the recipient of the prosthesis (20).

The prosthesis of Figure 4 includes a lubricant in the form of a glycerine based lubricant (24), which lubricates the cells (23) within the inner container (22).The inner layer (28) is effective in limiting the passage of the lubricant (24) from the prosthesis (20) to the body of a recipient thereof. It also limits the ingress of body fluids from the recipient into the prosthesis (20). The inner layer (28) therefore acts as an osmotic barrier which reduces the osmotic driving force which seeks to balance the concentrations of solutes on opposite sides of the barrier, in this case the shell (21), of the prosthesis (20).

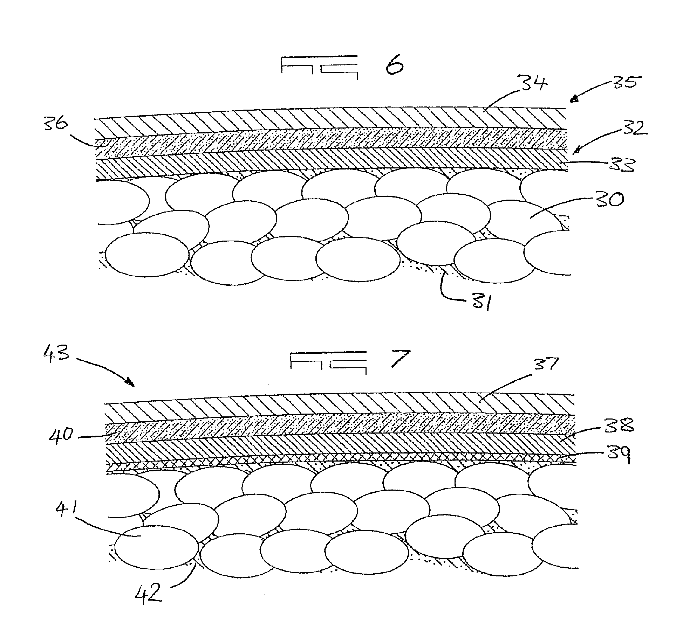

A second embodiment of a construction of inner and outer containers for a prosthesis according to the invention is shown in Figure 6. In this embodiment the prosthesis (not shown) includes a filler material in the form of a plurality of cells (30) in a polyvinyl pyrrolidone (PVP) based lubricant (31). The latter (31) acts as a lubricant for the cells (30) within the inner container (32). The PVP is a water soluble polymer and is hydrophilic, which means that it easily bonds with water by means of a hydrogen bond. In this way, the PVP is easily dissolved in water to form the lubricant (31) in the present instance.

The inner container (32) is formed by the inner layer (33), which comprises polyethylene. The PVP based lubricant (31) does not move through the polyethylene inner layer (33) under force of osmosis and the inner container (32) therefore provides an effective barrier to prevent loss of the lubricant (31) from the prosthesis.

Figure 6 shows the general construction of the layers of the prosthesis, namely the outer layer (34) which forms the shell (35) which contains the entire contents of the prosthesis. Below the outer layer (34) is the inner layer (33) which is separated from the outer layer (34) by a layer of lubricant (36), which is the same as the lubricant (31) used between the cells (30). The cells (30) within the PVP based lubricant (31) are located underneath the inner layer (33).

Figure 7 shows a third embodiment of a construction of inner and outer containers for a prosthesis according to the invention, which comprises an outer layer (37) similar to the outer layer (34) of Figure 6. Below this outer layer (37) there is an inner layer (38) comprised of polyethylene which carries a layer of aluminium (39). The aluminium layer (39) is adhered to the polyethylene inner layer (38) by means of a vapour deposition process. Between the inner layer 38() and the outer layer (37) there is a layer of lubricant (40) which is comprised of a mixture of glycerine and water. Below the inner layer (38) is shown the cells (41) within a lubricant (42), which is also comprised of a mixture of water and glycerine.

The laminate (43) shown in Figure 7 has a total thickness of about 48 micron, with the vapour deposited aluminium layer having a thickness in the range of about 12 micron to 18 micron. The total thickness of the laminate (43) may vary between about 20 micron and about 70 micron.

The embodiments of the laminates in combination with the respective lubricants shown in Figures 5 to 7 are effective in enclosing the cells and lubricant. The laminates and lubricants may be used in prostheses such as shown in Figure 4, i.e. a single outer container in the form of the silicone shell (21) and a single inner container (22).

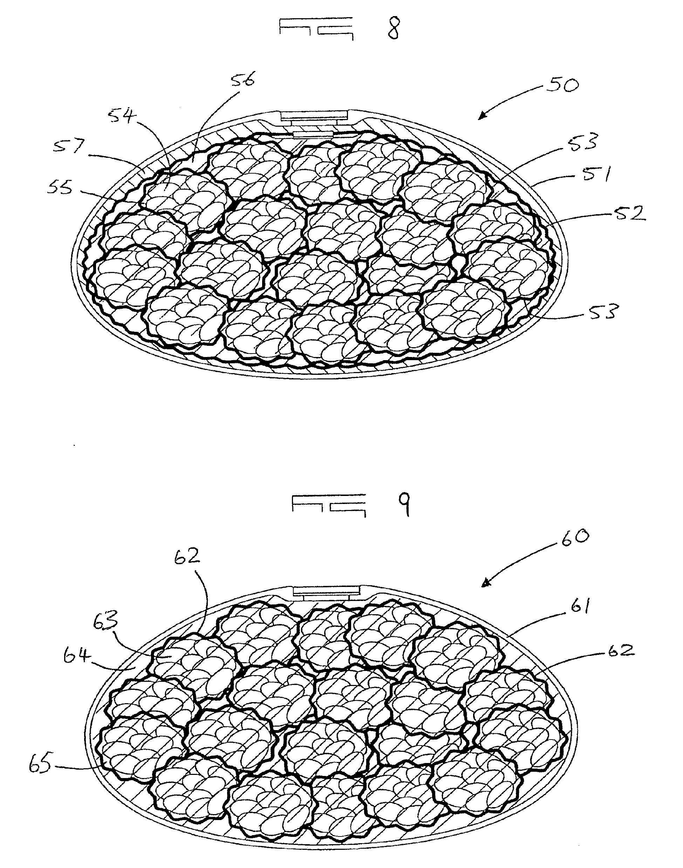

A third embodiment of a prosthesis (50) according to the invention in shown in Figure 8. In this embodiment the prosthesis (50) includes an outer container in the form of a silicone shell (51), an inner container (52) within which a plurality of small sachets (53) are contained. The inner container (52) and sachets (53) are manufactured from the laminates shown in any of Figures 5 to 7. Each of the sachets (53) contains a plurality of the biocompatible synthetic cells (54). Each sachet (53) includes lubricant (55) between the cells (54) and the inner container (52) includes lubricant (56) within which the sachets (53) are located. There is also lubricant (57) between the shell (51) and inner container (52).

The inner containers (22, 52) show in Figures 4 and 8 have surface areas which are greater than the surface area of their respective outer containers or shells (2, 51). In the embodiments shown in Figures 4 and 8 the respective surface areas of the inner containers (22, 52) are greater than the respective maximum surface area to which the respective outer containers or shells (2, 51) may be stretched elastically. This means, that the inner containers (22, 52) are large enough that any practical amount of elastic deformation which the shells (2, 51) of the prostheses may experience will not be so much that it will tear the inner containers (22, 52). In other preferred embodiments, the surface of the inner container should be at least twice as large as the maximum surface area to which the outer container may be stretched elastically.

A fourth embodiment of a prosthesis (60) according to the invention is shown in Figure 9. In this embodiment the prosthesis (60) includes an outer container in the form of a silicone shell (61), within which a plurality of small sachets (62) are contained. This embodiment does not include a single large inner container, but only a plurality of small sachets (62) within each of which there are a plurality of biocompatible synthetic cells (63). There is lubricant (64) within the shell (61) wherein the sachets (62) are located and each sachet (62) includes lubricant (65) between the cells (63).

The prosthesis shown in Figure 4 may be manufactured with an inner container from the laminate shown in Figure 6, which is a preferred embodiment of the invention. This embodiment includes an inner container which comprises a polyvinyl pyrrolidone (PVP) based lubricant and a plurality of the silicone cells, and having a polyvinyl pyrrolidone (PVP) based lubricant between the inner and outer containers.

In this embodiment the PVP based lubricant is mixed to comprise about 15% PVP in a saline solution. The prosthesis is manufactured in various sizes in three configurations, namely under filled, regularly filled, and over filled.

In the under filled configuration the prosthesis is filled to have a volume between 200 cm³ and 450 cm³ and to include the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 82.352 | 82.352 |
| 225 | 92.646 | 92.646 |
| 250 | 102.94 | 102.94 |
| 275 | 113.234 | 113.234 |
| 300 | 123.528 | 123.528 |
| 325 | 133.822 | 133.822 |
| 350 | 144.116 | 144.116 |
| 375 | 154.41 | 154.41 |
| 400 | 164.704 | 164.704 |
| 425 | 174.998 | 174.998 |
| 450 | 185.292 | 185.292 |

In the regularly filled configuration, the prosthesis is filled to have a volume between 200 cm³ and 450 cm³ and to include the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 87.5 | 87.5 |
| 225 | 98.4375 | 98.4375 |
| 250 | 109.375 | 109.375 |
| 275 | 120.3125 | 120.3125 |
| 300 | 131.25 | 131.25 |
| 325 | 142.1875 | 142.1875 |
| 350 | 153.125 | 153.125 |
| 375 | 164.0625 | 164.0625 |
| 400 | 175 | 175 |
| 425 | 185.9375 | 185.9375 |
| 450 | 196.875 | 196.875 |

In the over filled configuration, the prosthesis is filled to have a volume between 200 cm³ and 450 cm³ and to include the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 95 | 95 |
| 225 | 106.875 | 106.875 |
| 250 | 118.75 | 118.75 |
| 275 | 130.625 | 130.625 |
| 300 | 142.5 | 142.5 |
| 325 | 154.375 | 154.375 |
| 350 | 166.25 | 166.25 |
| 375 | 178.125 | 178.125 |
| 400 | 190 | 190 |
| 425 | 201.875 | 201.875 |
| 450 | 213.75 | 213.75 |

Trials indicate that prostheses which are under filled present with the best results, since it is almost impossible to discern the prosthesis from natural breast tissue in such a case.

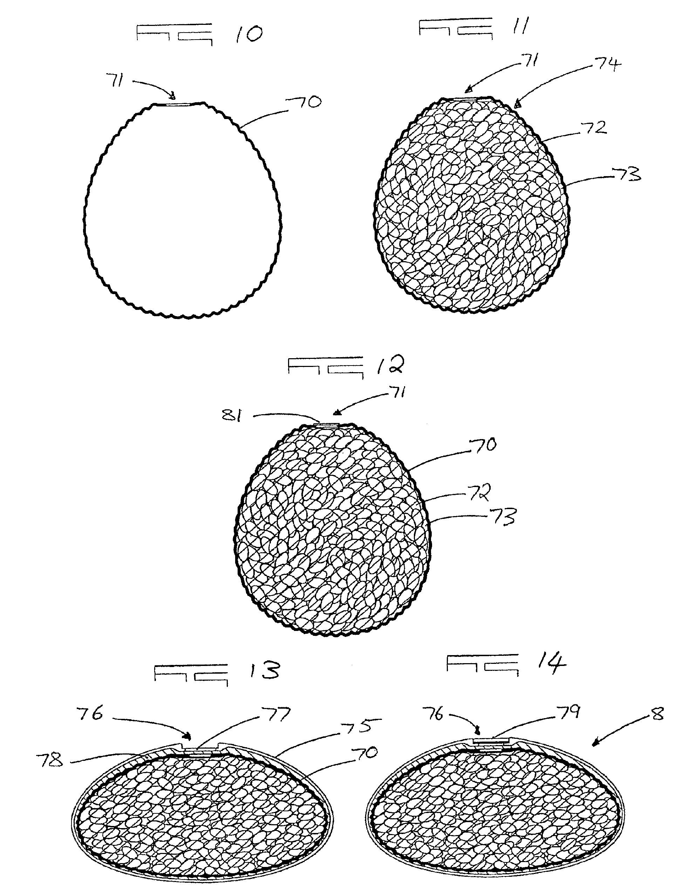

Figures 10 to 14 show the steps of one method of filling a prosthesis according to the invention.

As a first step, shown in Figure 10, a inner container (70) is formed from a laminate such as those shown in Figures 5 to 7. The inner container (70) includes a filling aperture (71) through which the correct weight of cells (72) and lubricant (73) is inserted. For a specific size prosthesis, these weights will correspond with the values given in the tables above, depending on whether the prosthesis is to be under filled, regularly filled or over filled.

Prior to inserting the filler material (74), i.e. the cells (72) and lubricant (73), into the inner container (70), the air inside the container (70) is evacuated using a vacuum pump. The filler material (74) is then inserted into the inner container (72) and it is sealed with a complimentary seal (81). The inner container (70) is then inserted into a pre-manufactured silicone shell (75). The filling aperture (76) of the shell (75) is closed with a membrane (77), which is a silicone disc which has a thickness typically of about 0.1 mm. Lubricant (78) is then injected through the membrane (77) by means of a needle (not shown), and the needle retracted. The membrane (77) is self sealing. The filling aperture (75) is then closed with a plug (79), which is a silicone disc with a thickness of about 0.9 mm. The plug (79) and membrane (77) create a seal of about 1 mm in the filling aperture. Both the membrane (77) and the plug (79) are secured to the shell (76) by means of silicone glue.

In this way the prosthesis (80) is filled with the required weight of cells (72) and lubricant (73, 78) and by using the vacuum process no excess air is left inside the prosthesis (80).

Once the prosthesis (80) has been filled it is sealed into a double layer package and the entire package is then subjected to radiation for a predetermined time, typically about 8 hours, to sterilize it. The package is only opened during the procedure to implant the prosthesis, which ensures that the prosthesis (80) remains sterile, at least until it is opened in the operating theatre.

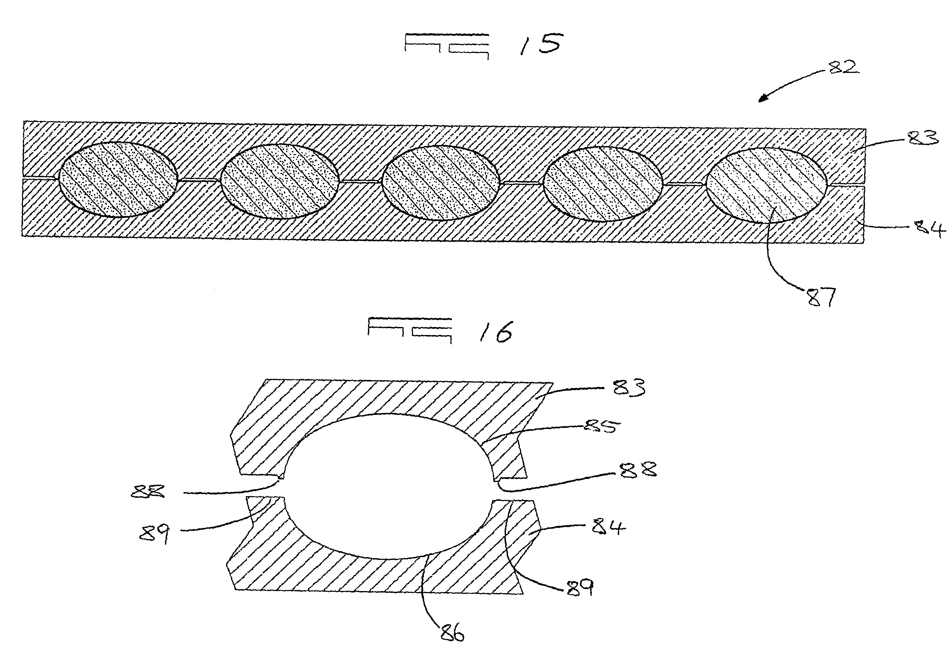

Figures 15 and 16 show a mould (82) used to manufacture the biocompatible synthetic cells used in the prostheses. The mould (82) comprises a set of two plates (83, 84) each of which includes a plurality of cavities (85, 86) of which each corresponds to a half-portion of a cell (87) formed therein. The plates (83, 84) may be aligned and closed to form the complete cell negative cavities (88) between them.

The process includes spraying the cavities (85, 86) on the plates (83, 84) with sterilized liquid silicone to a overfill the cavities (85, 86), aligning and closing the plates (83, 84). This forces the liquid silicone in the half-portion cavities (85, 86) to join to form a lenticular shaped silicone cell (87) between them. To aid the joining process heat may also be applied to the plates (83, 84) to the circular areas surrounding the cavities (85, 86).

After a predetermined time the liquid silicone sets and the plates (83, 84) are removed from each other to reveal the cells (87) formed between them.

Figure 16 shows a closer view of one of the cavities (85, 86) used to form the cells (87). The one plate cavity (85) includes an annular trimming formation (88) around the periphery of the cavity (85). When the plates (83, 84) are pressed close the trimming formation (88) bears against the flat surface (89) surrounding the opposing cavity (86) of the other plate (84) and trims the edge of the cell (87) where its two halves are joined.

It will be appreciated that the embodiments described above are not exhaustive of the invention and it is possible to alter some aspects of the prostheses without departing from the scope of the invention.

It is possible to use different sized or shaped biocompatible cells, for example ovoid or bean shaped cells.

It is also possible to use weights of cells and lubricant within the ranges of values shown in the tables.

It is further possible to include a tracer in the form of a particle in each cell to enhance the traceability of the cells. Titanium may for example be used to manufacture the particles. The particles will be inserted into the cells before forming them and will therefore form an inseparable part of each cell. In case of rupture of the shell and escape of some of the cells, a surgeon may use external diagnostic equipment or techniques including MRI, X-ray, or ultrasound to locate the exact position of each cell.

It is also possible to include a data transmission device, typically in the form of a transponder or Radio Frequency Identification tag, in the prosthesis. This RFID will be loaded with information relating to the prosthesis, including the size of the prosthesis, date of manufacture, and date of implanting into the patient. It may also be used to store the name of the patient, medical fund details of the patient, and emergency contact details relating to the patient. It is envisaged that this will allow a surgeon to establish the exact nature of the prosthesis without any invasive techniques. In the event of failure of the prosthesis, such as in a motor vehicle accident, where the patient may not be in position to convey critical information about the prosthesis an RFID scanner may be used to obtain information form the patient and perform any necessary procedure. This will also be helpful where a patient wishes to have a set of prostheses changed, for example for a larger set. In most instances, patients do not remember the details such as type, manufacturer and size of prosthesis. A surgeon then needs to have a range of prostheses on hand during the procedure to replace the prosthesis. If the surgeon knew the type of the original set of prostheses and their sizes, it becomes much easier and quicker to replace them. It is envisaged that the RFID tag will secured into one of the cells, or inside the shell. The surgeon will use a commercially available RFID scanner to energize the RFID tag, which triggers it to transmit the data stored on it. This data carrying signal is then received by the scanner and interpreted by custom software operated on the scanner. The information contained in the RFID tag is then displayed on a display screen of the scanner, or printed out.

In the same manner it will be possible to write information to the RFID tag. In this way the data stored on it may be updated, for example with the name of the patient, the date on which the prosthesis was implanted, the medical fund details of the patient and so forth.

It is also possible that the RFID tag may simply transmit a unique identification number and that the relevant details of the patient may be obtained from a secure computer server available on a network, typically the Internet. A physician will then log onto the secure server, enter the unique identification number obtained with the scanner and obtain information about the patient and the prosthesis from the server.

The present application is a divisional application of EP 06710272.3. The original claims of EP 06710272.3 are presented as statements below.

### STATEMENTS

1. A human prosthesis comprising a resilient shell at least partly filled with a biocompatible granular substance.
2. A prosthesis as in statement 1 which comprises a human mammary prosthesis.
3. A prosthesis as in statement 1 or statement 2 in which the granular substance comprises a plurality of biocompatible synthetic cells.
4. A prosthesis as in statement 3 in which the cells are manufactured from a resilient material.
5. A prosthesis as in statement 4 in which the resilient material comprises a medical grade silicone.
6. A prosthesis as in any one of statements 3 to 5 in which a plurality of micro glass balloons are dispersed in each cell.
7. A prosthesis as in statement 6 which includes between 2% and 10% micro glass balloons by volume.
8. A prosthesis as in statement 6 which includes between 4% and 8% micro glass balloons by volume.
9. A prosthesis as in statement 6 which includes between 5% and 7% micro glass balloons by volume.
10. A prosthesis as in statement 6 which includes about 6% micro glass balloons by volume
11. A prosthesis as in any one of statements 3 to 10 in which each cell has a lenticular shape.
12. A prosthesis as in any one of statements 3 to 10 in which each cell has an ovoid or a bean shape.
13. A prosthesis as in any one of statements 3 to 12 in which the cells have a range of sizes.
14. A prosthesis as in statement 11 in which each cell has a circular diameter of about 6 mm and a height of about 2 mm.
15. A prosthesis as in any one of statements 3 to 14 in which each cell comprises a hollow capsule encapsulating a fluid.
16. A prosthesis as in statement 15 in which the fluid comprises a biocompatible fluid.
17. A prosthesis as in statement 16 in which the biocompatible fluid comprises an oil.
18. A prosthesis as in statement 17 in which the oil comprises a vegetable oil.
19. A prosthesis as in statement 18 in which the vegetable oil comprises soy bean oil.
20. A prosthesis as in statement 16 in which the fluid comprises a gas.
21. A prosthesis as in statement 20 in which the gas comprises air.
22. A prosthesis as in statement 21 in which the air comprises sterilized air.
23. A prosthesis as in any one of statements 15 to 22 in which the cell has a wall thickness of about 0.1 mm.
24. A prosthesis as in any one of statements 3 to 23 in which each cell includes a tracer adapted to be detectable by means of external diagnostic equipment.
25. A prosthesis as in statement 24 in which the tracer comprises a metal particle contained in the cell.
26. A prosthesis as in statement 25 in which the metal particle comprises a titanium particle.
27. A prosthesis as in any one of statements 3 to 26 in which the cells are located within a lubricant.
28. A prosthesis as in statement 27 in which the lubricant is a biocompatible lubricant.
29. A prosthesis as in any one of statements 1 to 28 in which the shell includes a filling aperture which comprises a recessed portion in the shell within which a complimentary seal is securable to seal the bladder.
30. A prosthesis as in statement 29 in which the seal is securable to the shell by means of glue.
31. A prosthesis as in statement 30 in which the glue is silicone glue.
32. A prosthesis as in any one of statements 29 to 31 in which the seal comprises a membrane and a plug.
33. A prosthesis as in statement 32 in which the membrane is securable to the operative base of the recess and the plug is securable onto the membrane in the recess.
34. A prosthesis as in statement 32 or statement 33 in which the membrane has a thickness of about 0.1 mm and the plug has a thickness of about 0.9 mm.
35. A prosthesis as in any one of statements 29 to 34 in which the plug and the membrane are manufactured from the same material as the shell.
36. A prosthesis as in any one of statements 29 to 35 in which the plug and the membrane are manufactured from a medical grade silicone material.
37. A prosthesis as in any one of statements 29 to 36 in which the shell surrounding the recess is thickened such that the operatively inner surface of the shell inside of the aperture is substantially step-less.
38. A prosthesis as in any one of statements 29 to 37 in which the filler aperture and seal are circular.
39. A prosthesis as in any one of statements 1 to 38 in which the shell comprises at least one resilient layer and at least one layer being substantially non-permeable with respect to at least the filler material of the shell.
40. A prosthesis as in statement 39 in which the resilient layer comprises a sealed operatively outer container and the non-permeable layer comprises a sealed operatively inner container, the inner container contains the filler material, and the outer container contains the inner container.
41. A prosthesis as in statement 40 which includes a lubricant between the inner container and outer container.
42. A prosthesis as in statement 40 or 41 in which the surface area of the inner container is greater than the surface of the outer container.
43. A prosthesis as in statement 40 or 41 in which the surface area of the inner container is at least twice as great as the surface of the outer container.
44. A prosthesis as in statement 40 or 41 in which the surface area of the inner container is at least as great as the maximum size to which the outer container may be stretched elastically.
45. A prosthesis as in statement 39 in which the resilient layer comprises a sealed operatively outer container and the non-permeable layer comprises a plurality of sealed operatively inner containers, the inner containers contain the filler material, and the outer container contains the inner containers.
46. A prosthesis as in statement 45 which includes a lubricant between the inner container and outer containers.
47. A prosthesis as in statement 46 in which the non-permeable layer is non-permeable also with respect to human body fluids.
48. A prosthesis as in any one of statements 39 to 47 in which the non-permeable layer comprises a composite layer which includes a nylon layer located between a polyester and polyethylene layer.
49. A prosthesis as in statement 48 in which the lubricant within which the cells are located includes a hydrophilic polymer.
50. A prosthesis as in statement 49 in which the hydrophilic polymer comprises a water soluble polymer or hydrogel.
51. A prosthesis as in statement 50 in which the water soluble polymer or hydrogel comprises a polyvinyl pyrrolidone (PVP).
52. A prosthesis as in any one of statements 49 to 51 in which the hydrophilic polymer is mixed with a saline solution.
53. A prosthesis as in statement 52 in which the lubricant comprises about 15% hydrophilic polymer and about 85% saline solution.
54. A prosthesis as in any one of statements 39 to 47 in which the non-permeable layer comprises a composite polyethylene and aluminium layer.
55. A prosthesis as in statement 54 in which the aluminium layer comprises a vapour deposited layer of aluminium on the polyethylene layer.
56. A prosthesis as in statement 55 in which the aluminium layer has a thickness in the range of about 12 micron to 18 micron.
57. A prosthesis as in any one of statements 54 to 56 in which the lubricant includes glycerine.
58. A prosthesis as in statement 57 in which the lubricant comprises a combination of water and glycerine.
59. A prosthesis as in any one of statements 39 to 58 in which the non-permeable layer has a thickness of between about 20 micron and about 70 micron.
60. A prosthesis as in any one of statements 39 to 58 in which the non-permeable layer has a thickness of between about 40 micron and about 55 micron.
61. A prosthesis as in any one of statements 39 to 58 in which the non-permeable layer has a thickness of about 48 micron.
62. A prosthesis as in any one of the statements 1 to 38 in which the granular substance which fills the shell is contained in an inner container comprising a biocompatible resilient mesh.
63. A prosthesis as in any one of statements 1 to 62 which includes identification means bearing data relating to at least the size of the prosthesis, and for the identification means to include a remotely activatable data transmission device configured to transmit a predetermined data carrying signal in response to remote activation thereof.
64. A prosthesis as in statement 63 in which the transmission device comprises a data transponder which is remotely activatable by means of an energy field.
65. A prosthesis as in statement 64 in which the energy field comprises a magnetic field.
66. A prosthesis as in statement 64 or statement 65 in which the data transponder is incorporated into the shell.
67. A prosthesis as in statement 64 or statement 65 in which the data transponder is incorporated into a filler opening seal of the shell.
68. A prosthesis as in any one of statements 64 to 67 in which the data transponder comprises a Radio Frequency Identification (RFID) tag.
69. A prosthesis as in any one of statements 63 to 68 in which the data includes the number of cells contained within the prosthesis.
70. A prosthesis as in any one of statements 1 to 69 in which the filler material comprises between about 80% and 97% of the non-stretched volume of the shell.
71. A prosthesis as in statement 70 in which the filler material comprises between about 80% and 84% of the non-stretched volume of the shell.
72. A prosthesis as in statement 71 in which the filler material comprises about 82.3% of the non-stretched volume of the shell.
73. A prosthesis as in statement 70 in which the filler material comprises between about 85% and 90% of the non-stretched volume of the shell.
74. A prosthesis as in statement 73 in which the filler material comprises about 87.5% of the non-stretched volume of the shell.
75. A prosthesis as in statement 70 in which the filler material comprises between about 91 % and 97.5% of the non-stretched volume of the shell.
76. A prosthesis as in statement 75 in which the filler material comprises about 95% of the non-stretched volume of the shell.
77. A prosthesis as in any one of statements 3 to 76 in which the prosthesis is manufactured to a predetermined size and the prosthesis includes a predetermined number of cells.
78. A prosthesis as in any one of statements 3 to 76 in which the prosthesis is manufactured to a predetermined size and the prosthesis includes a predetermined weight of cells.
79. A prosthesis as in statement 53 in which a prosthesis having a volume between 200 cm³ and 450 cm³ includes the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 82.352 | 82.352 |
| 225 | 92.646 | 92.646 |
| 250 | 102.94 | 102.94 |
| 275 | 113.234 | 113.234 |
| 300 | 123.528 | 123.528 |
| 325 | 133.822 | 133.822 |
| 350 | 144.116 | 144.116 |
| 375 | 154.41 | 154.41 |
| 400 | 164.704 | 164.704 |
| 425 | 174.998 | 174.998 |
| 450 | 185.292 | 185.292 |

80. A prosthesis as in statement 53 in which a prosthesis having a volume between 200 cm³ and 450 cm³ includes the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 87.5 | 87.5 |
| 225 | 98.4375 | 98.4375 |
| 250 | 109.375 | 109.375 |
| 275 | 120.3125 | 120.3125 |
| 300 | 131.25 | 131.25 |
| 325 | 142.1875 | 142.1875 |
| 350 | 153.125 | 153.125 |
| 375 | 164.0625 | 164.0625 |
| 400 | 175 | 175 |
| 425 | 185.9375 | 185.9375 |
| 450 | 196.875 | 196.875 |

81. A prosthesis as in statement 53 in which a prosthesis having a volume between 200 cm³ and 450 cm³ includes the respective weight of cells and weight of polyvinyl pyrrolidone (PVP)/saline solution mixture in the inner container as shown in the table:

| Prosthesis volume (cm³) | Cells (g) | PVP mixture (g) |
|---|---|---|
| 200 | 95 | 95 |
| 225 | 106.875 | 106.875 |
| 250 | 118.75 | 118.75 |
| 275 | 130.625 | 130.625 |
| 300 | 142.5 | 142.5 |
| 325 | 154.375 | 154.375 |
| 350 | 166.25 | 166.25 |
| 375 | 178.125 | 178.125 |
| 400 | 190 | 190 |
| 425 | 201.875 | 201.875 |
| 450 | 213.75 | 213.75 |

82. A method of manufacturing cells, as in any one of statements 3 to 81, for use in a human mammary prosthesis which includes the steps of coating two plates, which each has a plurality of cavities therein and in which each cavity corresponds to a negative semi-portion of a cell, with a liquid resilient material, arranging the two plates together to align each individual cavity of one plate with another individual cavity on the other plate, allowing the resilient material to set, removing the plates from each other, and removing cells formed between the negative formations from the plates.
83. A method as in statement 81 in which a tracer is located within one semi-portion of each cell prior to arranging the plates together.
84. A method as in statement 82 or statement 83 which includes the step of trimming excess resilient material located around the circumference of each cell.
85. A method as in statement 84 in which the excess material is trimmed by integral trimming formations in at least one of the plates, each trimming formation surrounding a semi-portion cavity and being shaped and configured to press against the surface of the opposing plate upon aligning of the plates to trim excess resilient material from the formed cell.
86. A method of filling a shell of a human mammary prosthesis, as in any one of statements 3 to 38, with a filler material, which includes the steps of filling the shell with a predetermined number of biocompatible synthetic cells, securing a complimentary shaped membrane to an aperture in the shell, inserting a needle into the shell through the membrane, injecting a predetermined amount of a liquid biocompatible lubricant through the needle into the shell, retracting the needle, and sealing the filler aperture with a complimentary shaped seal.
87. A method as in statement 86 which includes evacuating substantially all air from the shell prior to injecting the lubricant into the shell.
88. A method of filling a shell of a human mammary prosthesis, as in any one of statements 3 to 61 and 63 to 78, with a filler material, which includes the steps of filling an inner container as in statement 40 with a predetermined number of biocompatible synthetic cells and a predetermined volume of liquid lubricant, sealing the inner container, inserting the inner container into an outer container as in statement 40, securing a complimentary shaped membrane to an aperture in the shell, inserting a needle into the shell through the membrane, injecting a predetermined amount of a liquid biocompatible lubricant through the needle into the shell, retracting the needle, and sealing the filler aperture with a complimentary shaped seal.
89. A method as in statement 88 which includes evacuating substantially all air from the shell prior to injecting the lubricant into the shell.
90. A prosthesis as in any one of the preceding statements in which the shell has a predetermined shaped corresponding to that of a breast.

## Claims

1. A human mammary prosthesis comprising a resilient shell at least partly filled with a plurality of resilient synthetic cells of which each cell has a lenticular shape.

2. A prosthesis as claimed in claim 1 in which the resilient shell and cells are comprised of a medical grade silicone.

3. A prosthesis as claimed in claim 2 in which each cell has a circular diameter of about 6 mm and a height of about 2 mm.

4. A prosthesis as claimed in any one of claims 1 to 3 in which each cell comprises a hollow capsule encapsulating a biocompatible fluid, the fluid preferably comprising an oil or a gas.

5. A prosthesis as claimed in claim 4 in which the oil comprises a vegetable oil, preferably soy bean oil.

6. A prosthesis as claimed in claim 5 in which the gas comprises air, preferably sterilized air.

7. A prosthesis as claimed in any one of claims 4 to 6 in which each hollow capsule has a wall thickness of about 0.1 mm.

8. A prosthesis as claimed in any one of claims 1 to 7 in which the cells are located within a biocompatible lubricant, preferably a hydrophilic polymer, more preferably a water soluble polymer or hydrogel.

9. A prosthesis as claimed in claim 8 in which the water soluble polymer or hydrogel comprises a polyvinyl pyrrolidone (PVP), which is preferably mixed with a saline solution.

10. A prosthesis as claimed in claim 9 in which the lubricant comprises about 15% hydrophilic polymer and about 85% saline solution.

11. A prosthesis as claimed in any one of the claims 1 to 10 in which the cells are contained in a closed inner container comprising a biocompatible resilient mesh, the mesh size being smaller than the diameter of a cell.

12. A prosthesis as claimed in any one of claims 1 to 11 in which the prosthesis is manufactured to a predetermined size which includes a predetermined number of cells.

13. A prosthesis as claimed in any one of claims 1 to 11 in which the prosthesis is manufactured to a predetermined size which includes a predetermined weight of cells.

14. A prosthesis as claimed in any one of claims 1 to 13 in which the shell includes a filling aperture which comprises a recessed portion in the shell within which a complimentary double lumen seal is securable, the seal comprises a membrane and a plug, the membrane is securable to the operative base of the recess and the plug is securable onto the membrane in the recess.

15. A prosthesis as claimed in claim 14 in which the membrane has a thickness of about 0.1 mm and the plug has a thickness of about 0.9 mm, and the shell surrounding the recess is thickened complimentary to the recess such that the operatively inner surface of the shell inside of the aperture is substantially step-less.
